Europäisches Patentamt

(19) European Patent Office

Office européen des brevets

(11) Numéro de publication: **0 313 426**
**A1**

(12) **DEMANDE DE BREVET EUROPEEN**

(21) Numéro de dépôt: 88402485.2

(22) Date de dépôt: 30.09.88

(51) Int. Cl.⁴: **A 61 F 13/18**

(30) Priorité: 20.10.87 FR 8714452

(43) Date de publication de la demande:
26.04.89 Bulletin 89/17

(84) Etats contractants désignés:
AT BE CH DE ES GB GR IT LI LU NL SE

(71) Demandeur: **Lebougault, Patrice**
**13, route de Nozay**
**F-10700 Arcis sur Aube (FR)**

**Madrières, Fabienne**
**13, route de Nozay**
**F-10700 Arcis sur Aube (FR)**

(72) Inventeur: **Lebougault, Patrice**
**13, route de Nozay**
**F-10700 Arcis sur Aube (FR)**

**Madrières, Fabienne**
**13, route de Nozay**
**F-10700 Arcis sur Aube (FR)**

(54) Article d'hygiène féminine, notamment serviette dite de protection périodique.

㊝ Article d'hygiène féminine comportant un matelas généralement constitué d'un matériau absorbant (7), dont une face, ou recto, est destinée à venir au contact corporel de l'utilisatrice et dont l'autre face, ou verso, est munie de moyens d'adhésion sur l'entrejambe d'un sous-vêtement.

Selon l'invention, il comporte, en position de stockage et en attente d'utilisation, deux volets séparés (3,4) d'un pan central (2) par des lignes de repli transversales, les deux volets d'extrémité étant repliés contre le pan central sans recouvrement d'un volet sur l'autre par leur verso, chacun des deux volets étant dimensionné de façon à venir en recouvrement sur la moitié adjacente du pan central, et audit pan central (2), est associée une contre-feuille intermédiaire d'adhésion (11) pourvue d'un produit adhésif sur chaque face (12,13).

Application aux articles d'hygiène féminine, notamment serviettes périodiques.

Fig. 2

# Description

## Article d'hygiène féminine, notamment serviette dite de protection périodique

La présente invention concerne un article d'hygiène féminine, notamment une serviette dite de protection périodique, permettant des utilisations associant un meilleur confort de l'utilisatrice et une efficacité accrue.

Les serviettes périodiques modernes sont maintenues en place au moyen de points d'adhérence qui sont prévus sur la face verso de celles-ci, et ces points d'adhérence sont destinés à permettre une solidarisation amovible de la serviette, par sa face verso, sur l'entrejambe du sous-vêtement de l'utilisatrice en assurant ainsi le maintien en place de la serviette pendant la période d'utilisation.

L'expérience montre cependant que la fixation sûre de la serviette sur le vêtement support est loin d'être obtenue dans des conditions satisfaisantes.

En effet, dans les dispositifs actuellement connus, l'adhérence de la face verso de la serviette sur le sous-vêtement est obtenue en prévoyant sur ladite face des zones pourvues de produit adhésif permettant ainsi cette solidarisation ; divers procédés de mise en place de ces zones adhésives ou diverses configurations géométriques pour la répartition et la définition de ces zones ont été prévus notamment par les brevets français publiés 2 526 309, 2 526 310 ou encore dans le brevet des Etats-Unis d'Amérique 3 672 371. Comme on le voit dans ces documents, notamment dans le brevet US 3 672 371, les zones adhésives prévues sur le verso sont protégées avant la période d'utilisation de la serviette par une contre-feuille de protection, contre-feuille qui est détachée ou "pelée" par l'utilisatrice en mettant ainsi à jour la zone adhésive, laquelle peut alors être rapportée et solidarisée sur l'entrejambe du sous-vêtement.

Il apparaît qu'une solidarisation plus sûre, plus efficace et donc plus confortable pour l'utilisatrice, serait obtenue si l'ensemble du verso de la serviette périodique pouvait être revêtu, sur l'ensemble de sa surface, d'un produit adhésif, permettant alors une solidarisation sûre de la serviette évitant toute distorsion par rapport au vêtement.

On comprend, en effet, qu'en cours d'utilisation et compte tenu des mouvements du sujet, la serviette soit soumise à des efforts et des sollicitations qui tendent à la refermer sur elle-même et à lui faire perdre sa position aussi largement étendue que possible pour offrir le maximum de surface absorbante au contact corporel de l'utilisatrice.

Et la limitation des points d'adhérence sur le sous-vêtement facilite par conséquent ce repli de la serviette sur elle-même et cette "compression" en largeur qui réduit l'efficacité de l'article en cours d'usage.

Cependant, l'enduction de l'ensemble du verso de la serviette par un revêtement d'un produit adhésif pose des problèmes notamment au niveau de la fabrication et entraînerait un coût de l'article prohibitif.

Notamment c'est l'ensemble de la surface de la serviette qui devrait recevoir une contre-feuille de protection ; cette contre-feuille réalisée généralement en papier siliconé représente un prix de revient non négligeable ; de sorte que cette solution n'a pu jusqu'à présent être retenue et mise en place.

Par ailleurs, il est d'usage que la serviette en cours de stockage et avant son utilisation soit repliée sur elle-même, deux volets d'extrémités étant rabattus l'un sur l'autre, dont l'un contre la partie centrale ; cette solution n'est pas satisfaisante dans la mesure où elle provoque en cours de stockage des replis transversaux sur la face absorbante, replis qui peuvent entraîner un disconfort lors de l'utilisation de la serviette.

La présente invention vise à remédier à ces divers inconvénients et concerne une serviette d'hygiène féminine de structure nouvelle et permettant des conditions fiables et efficaces d'utilisation en assurant le confort du sujet porteur.

Un premier objet de l'invention est de réaliser un article d'hygiène féminine permettant une adhérence optimale de sa surface sur le sous-vêtement, disposé en regard, de l'utilisatrice et ceci à partir d'une structure et d'une configuration nouvelles permettant la réalisation de cet article dans des conditions particulière ment économiques et avantageuses.

Un autre objet de l'invention est de permettre la réalisation d'un article d'hygiène féminine assurant la mise en place appropriée d'une surface d'absorption plus importante dans la partie centrale en épousant par conséquent la configuration anatomique de la zone où l'article doit remplir son office d'absorption.

Un troisième objet de l'invention est d'assurer la mise en place de l'article dans des conditions qui évitent tout phénomène de distorsion ou de repli de l'article sur lui-même en largeur et en assurant par conséquent le maintien en place de la zone centrale d'absorption, prévue plus importante, tout en évitant la déformation et la réduction en largeur de cette zone.

Enfin, un dernier objet de l'invention est d'éviter la formation, sur la face absorbante venant au contact corporel de l'utilisatrice, de plis transversaux liés aux replis pendant la période de stockage de l'article sur lui-même, par ladite face absorbante.

A cet effet, l'invention concerne un article d'hygiène féminine de structure générale longiforme et du type comportant un matelas généralement constitué d'un matériau absorbant, dont une face, ou recto, est destinée à venir au contact corporel de l'utilisatrice, et dont l'autre face, ou verso, est munie de moyens d'adhésion sur l'entrejambe d'un sous-vêtement, remarquable, selon l'invention, en ce qu'il comporte, en position de stocka ge et en attente d'utilisation, deux volets séparés d'un pan central par des lignes de repli transversales, les deux volets d'extrémité étant repliés contre le pan central sans recouvrement d'un volet sur l'autre par leur verso, chacun des deux volets étant dimensionné de façon à venir en recouvrement sur la moitié adjacente du pan central, et en ce que, audit pan central, est associée une contre-feuille intermédiaire d'adhésion pourvue d'un produit adhésif sur chaque face, de façon telle que cette contre-feuille adhère, de façon détachable, au verso dudit pan central, d'une part, et au verso de chacun des volets repliés contre elle, d'autre part, le déploiement des volets pour les amener en position d'alignement coplanaire avec le pan central (correspondant à la position d'utilisation) provoquant leur décollement de ladite contre-feuille intermédiaire, et ladite contre-feuille étant elle-même détachable dudit pan central de façon à abandonner sur le verso du pan central un dépôt de produit adhésif propre à permettre la solidarisation temporaire de l'article sur l'entrejambe du sous-vêtement.

Plus spécialement, dans l'article selon l'invention, ledit pan central est de largeur plus importante par rapport aux deux volets d'extrémité et les bords de cette partie centrale suivent une ligne curviforme définissant un renflement en largeur du pan central, l'ensemble ayant ainsi la forme d'un ovale prolongé longitudinalement par deux volets quadrangulaires. Cette forme permet d'éviter les fuites aux arêtes, et permet, par ailleurs, une bonne diffusion du liquide.

Notamment, la longueur des volets est sensiblement égale à la moitié de la longueur dudit pan central défini par la distance séparant les deux lignes de repli.

Chaque volet comporte, en outre, à son extrémité marginale un repli libre sans adhérence sur ledit pan central et permettant sa saisie par l'utilisatrice en vue du décollement dudit volet et du déploiement de l'ensemble amené en position coplanaire d'utilisation.

La contre-feuille intermédiaire est avantageusement rendue adhésive sur chaque face par un dépôt de produit adhésif permettant le détachement de ladite contre-feuille par rapport à son substrat, et la contre-feuille épouse sensiblement la surface du pan central lequel se trouve ainsi, après détachement de ladite contre-feuille, revêtu d'une couche d'adhésif sur au moins une partie de sa surface.

La contre-feuille comporte, en outre, une languette débordante en dehors dudit pan central permettant, de façon connue en soi, la saisie de cette contre-feuille et sa séparation du pan central, après déploiement des deux volets.

Enfin, et plus particulièrement dans le cadre de la réalisation de l'invention, l'ensemble du verso de l'article se trouve pourvu d'un revêtement de produit adhésif, après déploiement des deux volets et enlèvement de ladite contre-feuille centrale, le produit adhésif provenant par report de ladite contre-feuille, de sorte que ce revêtement permette une adhérence de toute la surface de l'article sur l'entrejambe du sous-vêtement.

Avantageusement, la contre-feuille intermédiaire est réalisée en papier siliconé.

Par ailleurs, le verso dudit matelas peut être revêtu d'un film en matière plastique imperméable, et au moins le recto dudit matelas peut être revêtu d'une feuille de protection, réalisée notamment en non-tissé.

D'autres caractéristiques et avantages de l'invention ressortiront de la description qui suit donnée en rapport avec les figures annexées, dans lesquelles :

- La figure 1 représente une vue en perspective d'un article d'hygiène féminine, en particulier une serviette, en cours de stockage et replié sur lui-même,
- la figure 2 représente une vue en coupe longitudinale de la serviette,
- la figure 3 représente une vue sensiblement en plan de la serviette de la figure 1,
- la figure 4 représente la serviette de la figure 1 en cours de déploiement des volets d'extrémité, tandis que
- la figure 5 représente la serviette dont les volets d'extrémité ont été déployés et amenés en position coplanaire avec le pan central,
- la figure 6 représente la serviette dont la contre-feuille centrale est en cours d'enlèvement pour mettre à nu le dépôt adhésif dudit pan central.

Selon l'ensemble des figures, on voit que la serviette 1 comporte un pan central 2 et deux volets d'extrémité 3 et 4.

Selon une caractéristique originale de l'invention, le pan central est lui-même de forme générale ovoïdale, les deux bords latéraux 5 et 6 dudit bord épousant un parcours curviforme procurant ainsi un renflement latéral donnant à l'ensemble de la serviette une zone centrale plus importante.

On offre ainsi dans cette zone qui correspond à la zone centrale d'utilisation et où le besoin d'absorption est maximum, une surface plus importante et ainsi anatomiquement mieux adaptée à son objet et à la morphologie du sujet.

La structure de la serviette est illustrée notamment à la figure 2.

On y voit que cette dernière se constitue, de façon connue en soi, d'un matelas 7, généralement constitué d'un matériau absorbant, par exemple en ouate de cellulose, qui est revêtu d'une enveloppe 8, 9, par exemple en non-tissé et dont le recto est destiné à venir au contact corporel de l'utilisatrice et dont le verso comporte un film imperméable 15.

La serviette est stockée à l'état replié, les deux volets 3 et 4 étant rapportés sur le pan central 2.

Mais de façon nouvelle, les deux volets 3 et 4 sont dimensionnés de façon à présenter une longueur sensiblement égale, pour chacun des volets, à la moitié de la longueur du pan central 2, de sorte que les volets viennent recouvrir ensemble le pan central, les extrémités des deux volets venant en juxtaposition sans recouvrement d'un volet sur l'autre.

On voit que, dans leur extrémité transversale marginale, les volets se terminent par un repli respectivement 10, 10′ en prolongement de l'enveloppe 8, 9, repli formé, par exemple, par scellage du bout des volets 3, 4 ; ces replis transversaux permettront, comme on le voit notamment à la figure 4, de saisir aisément les deux volets pour les séparer du pan central dans le mouvement de déploiement de la serviette en vue de l'amener dans sa position d'utilisation.

Au pan central 2 de forme ovale, est associée une contre-feuille 11, formée, par exemple, d'un papier siliconé, et revêtue sur ses deux faces d'un produit adhésif formant les couches de produit adhésif, respectivement inférieure 12 et supérieure 13.

Dans la position de stockage, on voit que les zones adhésives constituées des couches 12 et 13 qui peuvent correspondre sensiblement en surface, à l'ensemble de la surface du panneau central, permettent le maintien en position repliée des deux volets 3 et 4 contre la feuille 11 qui est elle-même maintenue, ainsi, solidaire du pan central 2, de sorte que l'ensemble forme un tout compacté.

Cela va permettre d'éviter la formation sur la face absorbante de lignes de repli susceptibles de former un bourrelet transversal gênant et désagréable lors de l'utilisation.

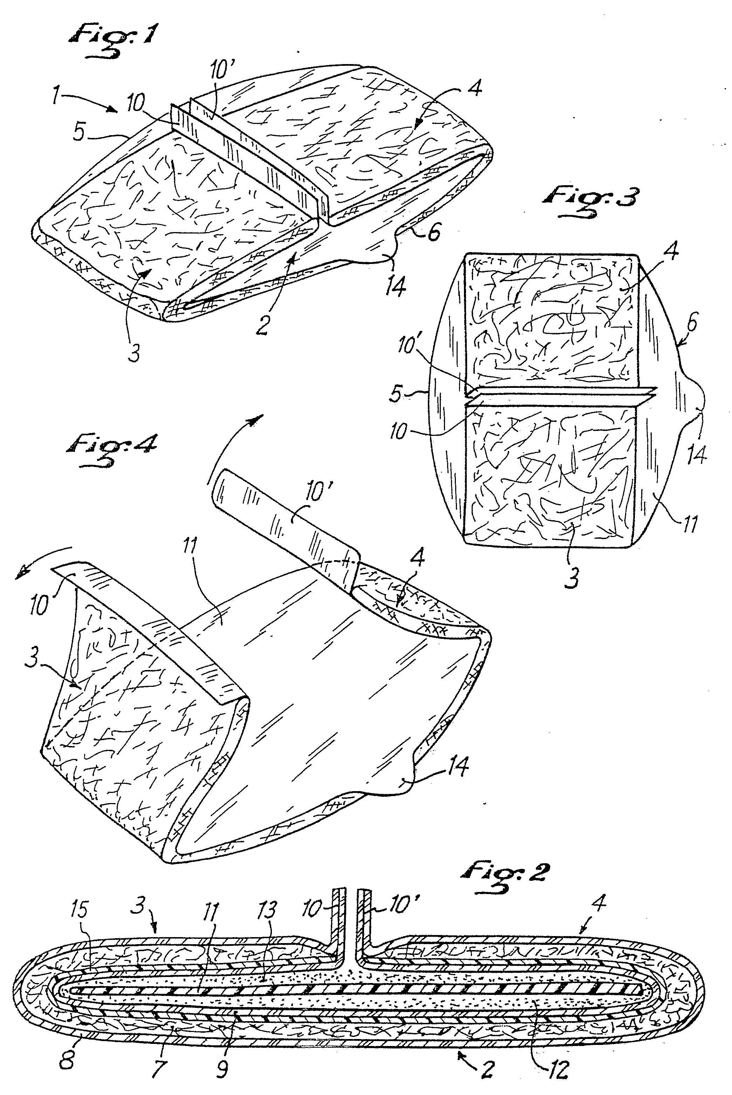

On conçoit, à la lumière des figures 4, 5 et 6, comment est mise en oeuvre la serviette selon l'invention, entre sa position de stockage repliée telle qu'elle est illustrée aux figures 1, 2 et 3 et la position d'utilisation.

Dans un premier temps, les volets 3 et 4 sont déployés par pivotement angulaire, l'utilisatrice pouvant aisément séparer ces volets du pan central en utilisant les plis marginaux 10 et 10', comme précédemment indiqué.

Dans ce mouvement, la couche adhésive 13 venue de la contre-feuille 11 reste en position contre l'enveloppe 9, la contre-feuille 11, par ses propriétés de non-adhérence, laissant ainsi, par report, le produit adhésif en position d'utilisation sur les versos des deux volets 3 et 4 qui vont ainsi recevoir un dépôt adhésif en position d'utilisation, comme on le voit sur la figure 5.

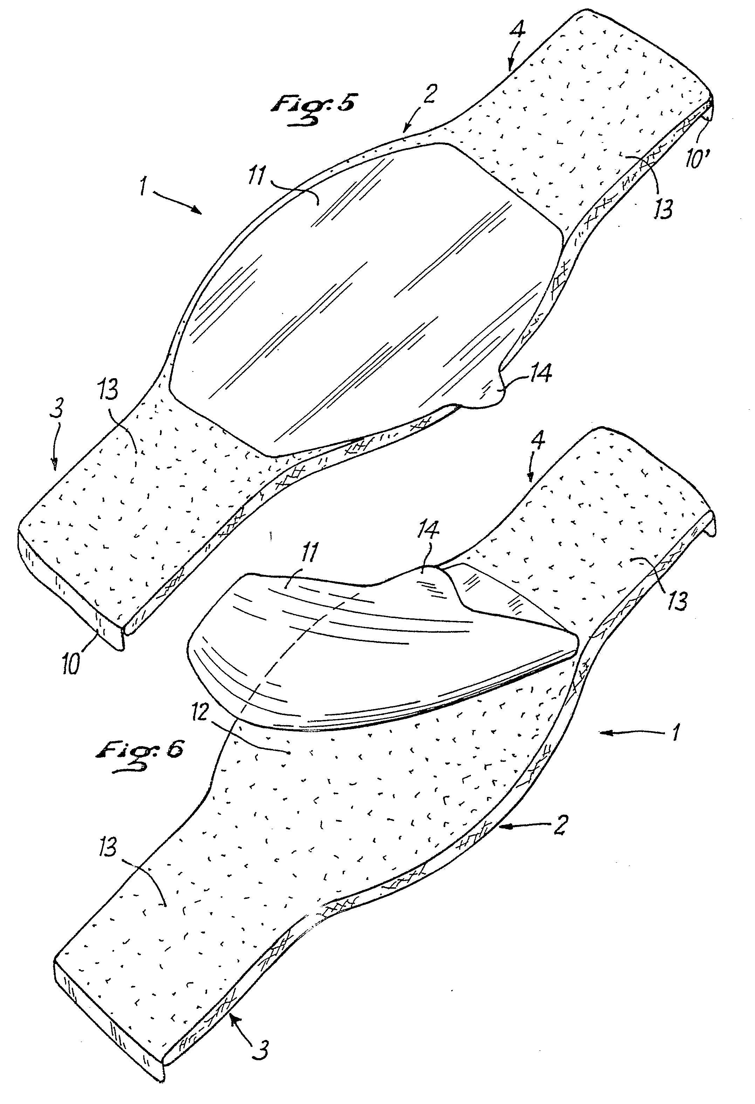

Par la suite, et comme on le voit sur la figure 6, il est aisé de saisir la contre-feuille 11 par sa languette latérale 14 et de peler cette feuille en la prélevant depuis le pan central 2, en dégageant ainsi le dépôt adhésif 12 et 13 qui recouvre sur son verso la serviette périodique.

On voit dès lors que cette serviette offre une surface revêtue de produit adhésif permettant ainsi une solidarisation sûre, fiable et confortable pour l'utilisatrice sur le sous-vêtement support.

Cependant on réalise que la mise en place de ce dépôt adhésif 12 et 13 se fait par des moyens très simples et économiques.

En effet, l'ensemble du dépôt adhésif 12 et 13 est rapporté en une seule fois au moyen de la contre-feuille 11 qui comporte un adhésif sur ses deux faces.

Comme on a prévu que les volets d'extrémité se rabattent par leur verso, c'est donc bien le verso qui venant au contact de la contre-feuille munie d'un produit adhésif, permet par conséquent, dans un premier temps d'assurer la tenue et l'homogénéité compactée de l'ensemble tandis que dans un deuxième temps, lors de la mise en position d'utilisation, le déploiement des volets amène automatiquement le report d'un dépôt adhésif sur la surface des volets.

La surface de la contre-feuille est limitée à la surface du pan central en réduisant par conséquent le coût de fabrication tant au niveau de la simplicité de mise en oeuvre, au niveau de la réalisation qu'en ce qui concerne le prix de revient des matières premières.

On voit donc que la mise en oeuvre selon une caracté ristique originale de l'invention d'une contre-feuille munie d'un produit adhésif sur ses deux faces et apte à abandonner son produit adhésif sur le verso de la serviette permet d'obtenir le résultat souhaité, à savoir, la mise en place, par simple report automatique, d'un dépôt adhésif sur tout ou partie de la surface formant le verso de la serviette.

On notera, par ailleurs, que la matière adhésive peut être mise en place, notamment au niveau du pan central, jusque sur les bords de ce pan central, de sorte que l'on évite les inconvénients des dispositifs antérieurs caractérisés par un report de matériau adhésif par zone, ce qui laissait subsister des zones dépourvues d'adhérence et entraînait, par conséquent, la formation de replis, de bourrelets ou un tassement de la serviette sur elle-même.

## Revendications

1 - Article d'hygiène féminine de structure générale longiforme et du type comportant un matelas généralement constitué d'un matériau absorbant (7), dont une face, ou recto, est destinée à venir au contact corporel de l'utilisatrice et dont l'autre face, ou verso, est munie de moyens d'adhésion sur l'entrejambe d'un sous-vêtement, caractérisé en ce qu'il comporte, en position de stockage et en attente d'utilisation, deux volets séparés (3,4) d'un pan central (2) par des lignes de repli transversales, les deux volets d'extrémité étant repliés contre le pan central sans recouvrement d'un volet sur l'autre par leur verso, chacun des deux volets étant dimensionné de façon à venir en recouvrement sur la moitié adjacente du pan central, et en ce que, audit pan central (2), est associée une contre-feuille intermédiaire d'adhésion (11) pourvue d'un produit adhésif sur chaque face (12,13), de façon telle que cette contre-feuille (11) adhère, de façon détachable, au verso dudit pan central (2), d'une part, et au verso de chacun des volets (3,4) repliés contre elle, d'autre part, le déploiement des volets pour les amener en position d'alignement coplanaire avec le pan central (correspondant à la position d'utilisation) provoquant leur décollement de ladite contre-feuille (11) intermédiaire, et ladite contre-feuille étant elle-même détachable dudit pan central de façon à abandonner sur le verso du pan central (2) un dépôt (12) de produit adhésif propre à permettre la solidarisation temporaire de l'article sur l'entrejambe du sous-vêtement.

2 - Article selon la revendication 1 ci-dessus, caractérisé en ce que ledit pan central (2) est de largeur plus importante par rapport aux deux volets (3,4) d'extrémité et les bords (5,6) de cette partie centrale suivent une ligne curviforme définissant un renflement en largeur du pan central, l'ensemble ayant ainsi la forme d'un ovale prolongé longitudinalement par deux volets quadrangulaires.

3 - Article selon l'une des revendications 1 ou 2 ci-dessus,
caractérisé en ce que la longueur des volets (3,4) est sensiblement égale à la moitié de la longueur dudit pan central (2) défini par la distance séparant les deux lignes de repli.

4 - Article selon l'une des revendications 1, 2 ou 3 ci,dessus,
caractérisé en ce que chaque volet (3,4) comporte à son extrémité marginale un repli libre (10,10') sans adhérence sur ledit pan central (2) et permettant sa saisie par l'utilisatrice en vue du décollement dudit volet et du déploiement de l'ensemble amené en position coplanaire d'utilisation.

5 - Article selon l'une des revendications 1 à 4 ci-dessus,
caractérisé en ce que la contre-feuille intermédiaire (11) est rendue adhésive sur chaque face (12,13) par un dépôt de produit adhésif permettant le détachement de ladite contre-feuille par rapport à son substrat, et la contre-feuille (11) épouse sensiblement la surface du pan central (2) lequel se trouve ainsi, après détachement de ladite contre-feuille, revêtu d'une couche d'adhésif sur au moins une partie de sa surface.

6 - Article selon l'une des revendications 1 à 5 ci-dessus,
caractérisé en ce que la contre-feuille comporte une languette débordante (14) en dehors dudit pan central (2) permettant, de façon connue en soi, la saisie de cette contre-feuille et sa séparation du pan central, après déploiement des deux volets.

7 - Serviette selon l'une des revendications 1 à 6, caractérisée en ce que l'ensemble du verso de l'article se trouve pourvu d'un revêtement (12,13) de produit adhésif, après déploiement des deux volets (3,4) et enlèvement de ladite contre-feuille centrale (11), le produit adhésif provenant par report de ladite contre-feuille (11), de sorte que ce revêtement (12,13) permette une adhérence de toute la surface de l'article sur l'entrejambe du sous-vêtement.

8 - Article selon l'une des revendications 1 à 7 ci-dessus,
caractérisé en ce que la contre-feuille intermédiaire (11) est réalisée en papier siliconé.

9 - Article selon l'une des revendications 1 à 8 ci-dessus,
caractérisé en ce que le verso dudit matelas (7) est revêtu d'un film en matière plastique imperméable (15).

10 - Article selon l'une des revendications 1 à 9 ci-dessus,
caractérisé en ce qu'au moins le recto dudit matelas (7) est revêtu d'une feuille de protection (8), réalisée notamment en non-tissé.

EP 0 313 426 A1

Fig.1

Fig.3

Fig.4

Fig.2

Fig. 5

Fig. 6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 229 639 (UNI-CHARM CORP.) <br> * Page 2, ligne 52 - page 3, ligne 53; figures * <br> --- | 1 | A 61 F   13/18 |
| A | EP-A-0 140 135 (KIMBERLY CLARK) <br> --- | | |
| A | EP-A-0 121 966 (SCHOOFS) <br> --- | | |
| D,A | FR-A-2 093 481 (KIMBERLY CLARK) <br> ----- | | |

DOMAINES TECHNIQUES RECHERCHES (Int. Cl.4)

A 61 F

Le présent rapport a été établi pour toutes les revendications

| Lieu de la recherche | Date d'achèvement de la recherche | Examinateur |
|---|---|---|
| LA HAYE | 19-01-1989 | STEENBAKKER J. |